# EUROPEAN PATENT APPLICATION

(11) **EP 3 712 137 A1**
(43) Date of publication of application: **23.09.2020**
(21) Application number: 19819092.8
(22) Date of filing: 11.06.2019
(51) Int. Cl.: C07D 249/12

(54) **METHOD FOR PRODUCING SOLID TRIAZOLINEDIONE COMPOUND, SOLID TRIAZOLINEDIONE COMPOUND, AND METHOD FOR PRODUCING TRIAZOLINEDIONE COMPOUND**

(30) Priority: 12.06.2018 JP 2018112192
(71) Applicant: Tokuyama Corporation, Shunan-shi, Yamaguchi 745-8648 (JP); Jeol Ltd., Akishima-shi, Tokyo 196-8558 (JP)
(72) Inventor: SEKI Masahiko, Shunan-shi, Yamaguchi 745-8648 (JP); FUKUZAWA Seketsu, Akishima-shi, Tokyo 196-8558 (JP); TAKIWAKI Masaki, Akishima-shi, Tokyo 196-8558 (JP)
(74) Representative: Jones, Nicholas Andrew
(86) International application number: PCT/JP2019/023163
(87) International publication number: WO 2019/240140

(57) **Abstract**

Provided are a method for separating a DAPTAD-containing triazolinedione compound in solid form from a reaction solution, a separated solid triazolinedione compound, and a novel method for producing a triazolinedione compound. A triazolinedione solution in which a DAPTAD-containing triazolinedione compound is dissolved is brought into contact with a C5-15 hydrocarbon-based poor solvent to obtain a solid triazolinedione compound. Also, a triazolinedione compound is oxidized using an oxidizing agent that does not produce acid as a byproduct to obtain a triazolinedione compound.

## Description

### TECHNICAL FIELD

The present invention relates to a method for producing a solid triazolinedione compound, a solid triazolinedione compound, and a method for producing a triazolinedione compound.

### BACKGROUND ART

In recent years, analysis of vitamin D and vitamin D metabolites in blood has been required. Vitamin D promotes the absorption of calcium and phosphorus in the body, maintains the concentration of calcium in the blood, and helps form strong bones. Vitamin D metabolites are also known to be involved in the control of expression of proteins involved in cell differentiation and proliferation, hormone production and secretion as well as immune responses, etc.

However, since the vitamin D metabolite in the blood is a trace component, there is a problem that the measurement sensitivity is insufficient. Therefore, there has been proposed a method of derivatization using PTAD (4-phenyl-1,2,4-triazoline-3,5-dione), etc., which is a Cookson type derivatization reagent, and then analyzing the derivatives (see Patent Document 1).

DAPTAD (4-(4'-dimethylaminophenyl)-1,2,4-triazoline-3,5-dione) has been proposed as a novel Cookson-type derivatization reagent that can further improve the sensitivity (see Non-Patent Documents 1 to 4).

Vitamin D derivatized with DAPTAD is about 100 times more sensitive than vitamin D prior to derivatization and about 10 times more sensitive than conventional PTADs. In addition, since the derivatives by DAPTAD can be quantitatively determined by distinguishing structural isomers, selectivity can be further improved.

Patent Document 1: Japanese Unexamined Patent Application, Publication No. 2015-166740
Non-Patent Document 1: S. Ogawa, et al., Rapid Commun. Mass Spectrom, 27(2013) 2453-2460
Non-Patent Document 2: S. Ogawa, et al., Biomed. Chromatgr., 30(2016) 938-945
Non-Patent Document 3: S. Ogawa, et al., J. Pharm. Biomed. Anal., 136(2017) 126-133
Non-Patent Document 4: K.D. Bruycker, et al., Chem. Rev., 116(2016) 3919-3974

### DISCLOSURE OF THE INVENTION

### Problems to be Solved by the Invention

DAPTAD is known to be obtained by oxidatively reacting 4-(4'-dimethylaminophenyl)-1,2,4-triazolidine-3,5-dione (DMU) with iodobenzene diacetate (PIDA) in ethyl acetate (see Non-Patent Documents 1 to 4).

However, it was difficult to isolate DAPTAD from the obtained reaction solution (see, for example, page 2456 of Non-Patent Document 1, lines 2 to 4 in the right column), and DAPTAD was used for the subsequent reaction while being dissolved in the reaction solution. Therefore, methods for isolating DAPTAD as crystals and the isolated DAPTAD itself have not yet been known.

In addition, according to trial and error by the present inventors, a synthetic method in which an oxidative reaction is performed using PIDA involved possibility that acetic acid is simultaneously generated as a by-product along with the production of DAPTAD, and the acetic acid produced as a by-product decomposes DAPTAD. On the other hand, it has been found that when the DAPTAD is washed with an alkali aqueous solution in order to remove the by-product acetic acid, the DAPTAD is also decomposed by the alkali.

The present invention has been made in view of the above-mentioned background art, and an object of the present invention is to provide a method for isolating a triazolinedione compound including DAPTAD in a solid state from a reaction solution and a solid triazolinedione compound as well as a novel method for producing a triazolinedione compound.

### Means for Solving the Problems

The present inventors have found that a triazolinedione compound can be isolated as crystals by contacting a solution in which a triazolinedione compound including DAPTAD is dissolved with a specific solvent, and have thus completed the present invention.

The present inventors have focused on a fact that if oxidation is performed using an oxidizing agent that does not generate acid as a by-product in obtaining a triazolinedione compound by oxidation of a triazolidinedione compound, decomposition of the produced triazolinedione compound can be prevented, and have thus completed the present invention.

Namely, the present invention is a method for producing a solid triazolinedione compound including a step of contacting a triazolinedione solution in which a triazolinedione compound represented by the following formula (1) is dissolved in an aprotic good solvent with a hydrocarbon-based poor solvent having 5 to 15 carbon atoms in a light-shielded condition at -25 to 30°C: 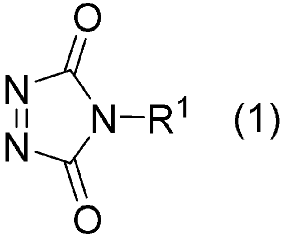 wherein R¹ is an organic group.

The aprotic good solvent may be at least one selected from the group consisting of esters, halogen-containing hydrocarbons, aromatic hydrocarbons, ketones, amides, alkylnitriles, dialkyl ethers and ureas.

In the contacting step, a triazolinedione solution and a hydrocarbon-based poor solvent may be brought into contact with each other by setting an amount of the aprotic good solvent in the triazolinedione solution to from 100 parts by mass to 200,000 parts by mass relative to 100 parts by mass of the triazolinedione compound represented by the formula (1); setting an amount of the hydrocarbon-based poor solvent to from 2,500 to 500,000 parts by mass relative to 100 parts by mass of the triazolinedione compound represented by the formula (1); and setting a mass ratio of the aprotic good solvent to the hydrocarbon-based poor solvent to 1:0.05 to 1:10.

The triazolinedione solution may be obtained by an oxidation step in which a triazolidinedione compound represented by the following formula (2) is oxidized using an oxidizing agent that does not generate acid as a by-product to obtain the triazolinedione compound represented by the formula (1) : 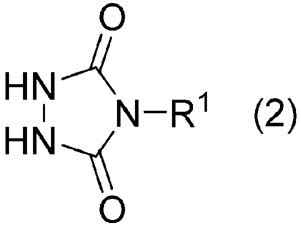 wherein R¹ is an organic group.

The oxide may be an iodosobenzene.

The oxidation step may be performed while removing water produced as a by-product.

The oxidation step may be performed in a light-shielded condition.

Another aspect of the present invention is a solid triazolinedione compound represented by the following formula (1) : 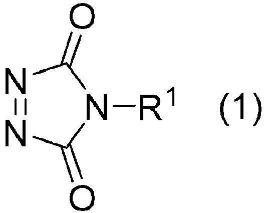 wherein R¹ is a group selected from the group consisting of (a), (b) and (c) below:
(a) a substituted phenyl group that includes a disubstituted amino group or a disubstituted aminoalkyl group, with the disubstituted amino group or the disubstituted aminoalkyl group being substituted with the same or different alkyl groups, aralkyl groups or aryl groups, with the alkyl groups, the aralkyl groups or the aryl groups optionally containing an oxygen atom or a nitrogen atom; a nitro group; an azide group, an alkoxy group; a halogen group; an alkylthio group; a sulfonyl group, a phosphate group; a carboxyl group; an ester group; a nitrile group; an amide group; a ferrocenyl group or a substituted quinoxalinyl group;
(b) a nitrogen-containing heterocyclic group that may include a disubstituted amino group substituted with the same or different alkyl groups, aralkyl groups or aryl groups, with the alkyl groups, the aralkyl groups or the aryl groups optionally containing an oxygen atom or a nitrogen atom; a nitro group; an azide group; an alkoxy group; a halogen group; an alkylthio group; a sulfonyl group; a phosphate group; a carboxyl group; an ester group; a nitrile group; an amide group; a ferrocenyl group or a substituted quinoxalinyl group; and
(c) an alkyl group that may include a disubstituted amino group substituted with the same or different alkyl groups, aralkyl groups or aryl groups, with the alkyl groups, the aralkyl groups or the aryl groups optionally containing an oxygen atom or a nitrogen atom; a nitro group; an azide group; an alkoxy group; a halogen group; an alkylthio group; a sulfonyl group; a phosphate group; a carboxyl group; an ester group; a nitrile group; an amide group; a ferrocenyl group or a substituted quinoxalinyl group.

R¹ in the formula (1) may be a 4-dimethylaminophenyl group or a 4-dimethylaminomethylphenyl group.

Another aspect of the present invention is a method for producing a triazolinedione compound, the method comprising oxidizing a triazolidinedione compound to obtain a triazolinedione compound, in which the triazolidinedione compound represented by the following formula (2) is oxidized by using an oxidizing agent that does not generate acid as a by-product to obtain a triazolinedione compound represented by the following formula (1): 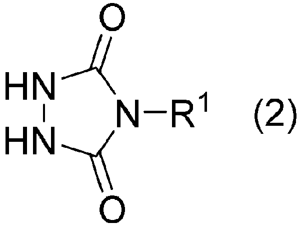 wherein R¹ is an organic group, 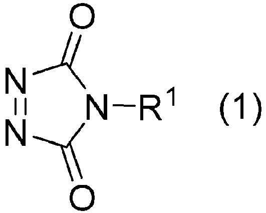 wherein R¹ is an organic group.

### Effects of the Invention

According to the present invention, a triazolinedione compound which has not been previously isolated can be isolated in a solid state as crystals. The resulting solid triazolinedione compound is stable because it can be stored as a solid. In addition, since solvent, which is required when storing as a solution, is unnecessary, there is no risk of ignition or the like due to exposure of the solvent, and handling becomes easier. Further, since the triazolinedione compound could not be isolated conventionally, the component of the solvent contained in the reaction solution had influence. However, the present invention enables a user to freely select a solvent. As a result, the application environment of the triazolinedione compound is remarkably expanded, and the triazolinedione compound is expected to be used not only in existing applications but also in the development of new applications.

### PREFERRED MODE FOR CARRYING OUT THE INVENTION

Embodiments of the present invention are described below. It should be noted that the embodiments described below do not limit the present invention.

### <Method for producing Solid Triazolinedione Compound>

The inventive method for producing a solid triazolinedione compound includes a step of contacting a triazolinedione solution in which the triazolinedione compound represented by the following formula (1) is dissolved in an aprotic good solvent, with a hydrocarbon-based poor solvent having 5 to 15 carbon atoms in a light-shielded condition at -25 to 30°C to obtain the solid triazolinedione compound: 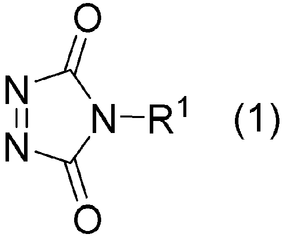 wherein R¹ is an organic group.

The present invention enables triazolinedione compounds, such as 4-(4'-dimethylaminophenyl)-1,2,4-triazoline-3,5-dione (DAPTAD), which have not been conventionally isolated, to be isolated in a solid state as crystals.

### [Triazolinedione compounds]

The solid triazolinedione compound of the present invention obtained by the production method of the present invention is a compound having a structure represented by the formula (1). Here, R¹ in the formula (1) is a group selected from the group consisting of (a), (b) and (c) below:
(a) a substituted phenyl group that includes a disubstituted amino group or a disubstituted aminoalkyl group, with the disubstituted amino group or the disubstituted aminoalkyl group being substituted with the same or different alkyl groups, aralkyl groups or aryl groups, with the alkyl groups, the aralkyl groups or the aryl groups optionally containing an oxygen atom or a nitrogen atom; a nitro group; an azide group, an alkoxy group; a halogen group; an alkylthio group; a sulfonyl group, a phosphate group; a carboxyl group; an ester group; a nitrile group; an amide group; a ferrocenyl group or a substituted quinoxalinyl group;
(b) a nitrogen-containing heterocyclic group that may include a disubstituted amino group substituted with the same or different alkyl groups, aralkyl groups or aryl groups, with the alkyl groups, the aralkyl groups or the aryl groups optionally containing an oxygen atom or a nitrogen atom; a nitro group; an azide group; an alkoxy group; a halogen group; an alkylthio group; a sulfonyl group; a phosphate group; a carboxyl group; an ester group; a nitrile group; an amide group; a ferrocenyl group or a substituted quinoxalinyl group; and
(c) an alkyl group that may include a disubstituted amino group substituted with the same or different alkyl groups, aralkyl groups or aryl groups, with the alkyl groups, the aralkyl groups or the aryl groups optionally containing an oxygen atom or a nitrogen atom; a nitro group; an azide group; an alkoxy group; a halogen group; an alkylthio group; a sulfonyl group; a phosphate group; a carboxyl group; an ester group; a nitrile group; an amide group; a ferrocenyl group or a substituted quinoxalinyl group.

More preferably, R¹ in the formula (1) is a group selected from the group consisting of substituted phenyl groups and substituted or unsubstituted methyl and ethyl groups.

Further, it is particularly preferable that R¹ in the formula (1) is a group selected from the group consisting of a methyl group, a 2-(6,7-dimethoxy-4-methyl-3-oxo-3,4-dihydroquinoxalinyl) ethyl group, a 4-nitrophenyl group, a ferrocenyl methyl group, a 4-dimethylaminophenyl group and a 4-dimethylaminomethylphenyl group.

Namely, as the compound to which the inventive method for producing a solid triazolinedione compound represented by the formula (1) can be applied, and which can be converted to the solid triazolinedione compound of the present invention, a compound selected from the group consisting of 4-methyl-1,2,4-triazoline-3,5-dione (MTAD), 4-[2-(6,7-dimethoxy-4-methyl-3-oxo-3,4-dihydroquinoxalinyl)ethyl]-1,2,4-triazoline-3,5-dione (DMEQTAD), 4-(4-nitrophenyl)-1,2,4-triazoline-3,5-dione (NPTAD), 4-ferrocenylmethyl-1,2,4-triazoline-3,5-dione (FMTAD), 4-(4'-dimethylamino phenyl)-1,2,4-triazoline-3,5-dione (DAPTAD) and 4-(4'-dimethylaminomethylphenyl)-1,2,4-triazoline-3,5-dione is particularly preferred. These compounds may be Cookson type derivatizing agents.

Furthermore, examples of the most preferred R¹ in the formula (1) include a 4-dimethylaminophenyl group or a 4-dimethylaminomethylphenyl group. Namely, examples of the most preferred compound to which the inventive method for producing a solid triazolinedione compound can be applied, and which can be converted to the solid triazolinedione compound of the present invention include 4-(4'-dimethylaminophenyl)-1,2,4-triazoline-3,5-dione (DAPTAD) or 4-(4'-dimethylaminomethylphenyl)-1,2,4-triazoline-3,5-dione.

The crystalline structure and purity of the solid triazolinedione compound of the present invention obtained by the production method of the present invention can be confirmed by infrared spectroscopy (IR), ultraviolet spectroscopy (UV) or ¹H-NMR analysis and the like. The storage temperature of the solid triazolinedione compound of the present invention obtained by the production method of the present invention is suitably a low temperature of -20°C or less.

### [Reaction scheme]

A representative reaction scheme of the inventive production method of a solid triazolinedione compound is indicated below. It should be noted that the following is an example of the present invention, and the present invention is not limited to the following.

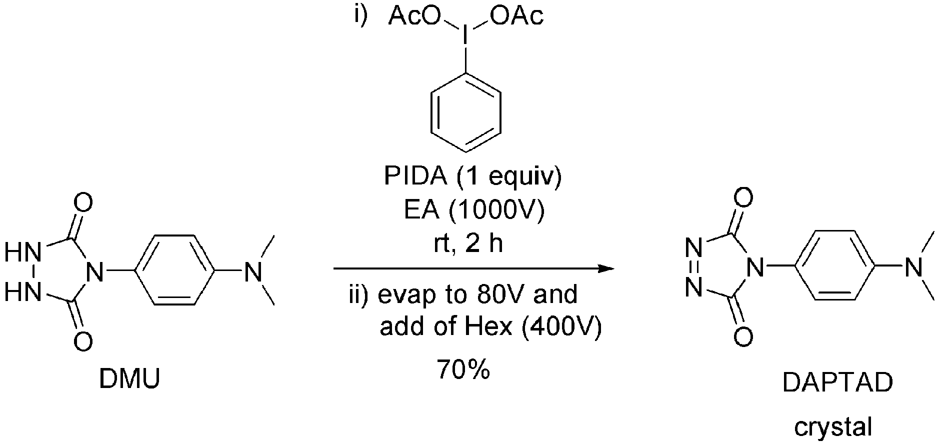

### [Triazolinedione solution]

The triazolinedione solution used in the inventive method for producing a solid triazolinedione compound is a solution in which a triazolinedione compound represented by the following formula (1) is dissolved in an aprotic good solvent.

The method of obtaining the triazolinedione solution is not particularly limited, and a method can be mentioned such that 4-(4'-dimethylaminophenyl)-1,2,4-triazolidine-3,5-dione (DMU) is oxidized in ethyl acetate (EA) with iodobenzene diacetate (PIDA) and the obtained reaction solution is used as a triazolinedione solution, for example, as shown in i) in the above reaction scheme.

Namely, in the present invention, it is also possible to carry out an oxidation step of obtaining a triazolinedione compound by oxidizing a triazolidinedione compound with an oxidizing agent and use the obtained reaction solution as it is, as a triazolinedione solution.

When a reaction solution obtained by the oxidation step of a triazolidinedione compound is used as the triazolinedione solution, it is preferable that the triazolidinedione compound which is a raw material be a compound containing a urazole group represented by the following formula (2). Namely, a compound having a 1,2,4-triazolidine-3,5-dione group is preferable: 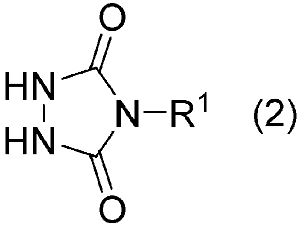 wherein R¹ is an organic group.

Note that R¹ in the formula (2) is the same group as R¹ in the formula (1).

### (Aprotic Good Solvent)

The aprotic good solvent for dissolving a triazolinedione compound is preferably at least one type selected from the group consisting of esters, halogen-containing hydrocarbons, aromatic hydrocarbons, ketones, amides, alkylnitriles, dialkyl ethers and ureas.

Here, the "aprotic good solvent" in the present invention refers to a solvent which does not contain a hydrogen atom which is easily dissociated, which easily dissolves a solid, and in which a triazolinedione compound has a solubility of 0.001 g/100 mL or more.

Examples thereof include ethyl acetate, methyl acetate, butyl acetate, isopropyl acetate, tetrahydrofuran (THF), 2-methyltetrahydrofuran (2-methyl THF), 1,4-dioxane, t-butyl methyl ether, 1,2-dimethoxyethane, diglyme, acetone, diethyl ketone, methyl ethyl ketone, methylene chloride, chloroform, carbon tetrachloride, 1,2-dichloroethane, chlorobenzene, toluene, xylene, dimethylformamide (DMF), dimethylacetamide (DMA), dimethylsulfoxide (DMSO), N-methylpyrrolidone (NMP) and 1,3-dimethyl-2-imidazolidinone (DMI), etc. These may be used singly or as mixed solvents. Nitrogen bubbling may be performed to remove dissolved oxygen.

Among these, it is preferable to use at least one type selected from the group consisting of methylene chloride, acetonitrile, 1,2-dimethoxyethane, chlorobenzene, toluene and ethyl acetate in the inventive production method of a solid triazolinedione compound, from a viewpoint of stabilization of the triazolinedione compound to be produced.

### (Oxidizing agent)

When a reaction solution obtained by the oxidation step of a triazolidinedione compound is used as the triazolinedione solution, iodobenzene diacetate (PIDA) shown in, for example, i) in the above reaction scheme, is known as an oxidizing agent (see Non-Patent Documents 1 to 4).

Here, as the oxidizing agent for oxidizing the triazolidinedione compound, for example, a super-valent iodine compound represented by the following formula can be mentioned. The above-mentioned iodobenzene diacetate (PIDA) is an example of the super-valent iodine compound represented by the following formula: 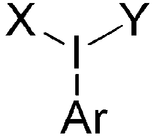 wherein X and Y, which are the same as or different from each other, represent groups selected from the group consisting of a hydroxy group, an alkoxy group, an acyloxy group, an acylamino group, a tosylamino group, a mesylamino group, a sulfonyloxy and a halogen group; and Ar represents a group selected from the group consisting of a phenyl group, heterocyclic groups and phenyl groups substituted with an alkyl group, an alkoxy group or a halogen group, etc.

Further, in the inventive method for producing a solid triazolinedione compound, when a reaction solution obtained by the oxidation step of a triazolidinedione compound is used as the triazolinedione solution, it is preferable that the triazolidinedione compound represented by the formula (2) be oxidized using an oxidizing agent that does not generate acid as a by-product and the obtained reaction solution be used as a reaction solution which has undergone the oxidation step for obtaining the triazolinedione compound represented by the formula (1).

For example, when a triazolidinedione compound is oxidized by iodobenzene diacetate (PIDA) described in Non-Patent Documents 1 to 4, which is conventionally known as an oxidizing agent, acetic acid is generated as a by-product. This by-product acetic acid may decompose the formed triazolinedione compound. On the other hand, when washing with an alkali aqueous solution is performed in order to remove the by-product acetic acid, the triazolinedione compound is also decomposed by the alkali.

Therefore, when a reaction solution obtained through the oxidation step of a triazolidinedione compound is used as the triazolinedione solution, it is preferable to use as the oxidizing agent an oxidizing agent which does not generate by-product acid.

Examples of the oxidizing agent that does not generate acid as a by-product include the above-mentioned hyper-valent iodine compound, in which X and Y together form one oxygen atom, or X and Y are the same or different groups selected from the group consisting of a hydroxy group, an alkoxy group, an acylamino group and a tosylamino group.

In the present invention it is preferable to use, for example, iodosobenzene (PIO) described in the following formula.

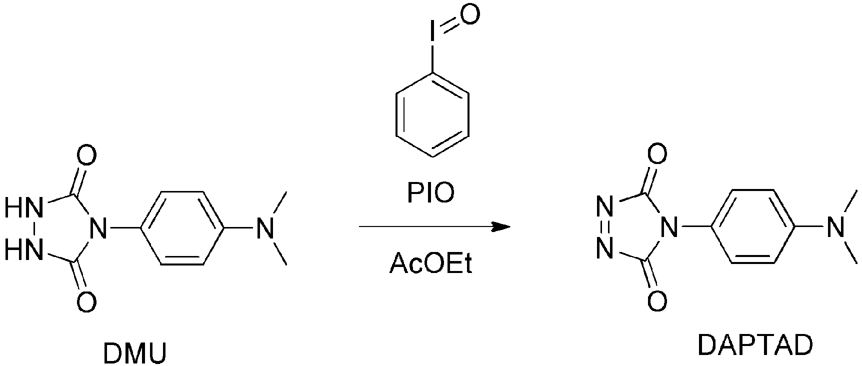

The amount of the oxidizing agent used is preferably 1.0 to 5.0 mol equivalents, more preferably 1.0 to 2.0 mol equivalents, with respect to the triazolidinedione compound as a raw material. The range of 1.0 to 5.0 molar equivalents is preferable because it facilitates removal of the oxidizing agent present after the reaction and of decomposition products thereof.

### (Oxidation Conditions)

When a reaction solution obtained by the oxidation step of a triazolidinedione compound is used as the triazolinedione solution, oxidation temperatures are preferably in the range of -10 to 50°C, more preferably in the range of 0 to 30°C.

When a reaction solution obtained by the oxidation step of a triazolidinedione compound is used as the triazolinedione solution, reaction time for oxidation is preferably 1 minute to 48 hours, more preferably 10 minutes to 17 hours.

Additionally, when a reaction solution obtained by the oxidation step of a triazolidinedione compound is used as the triazolinedione solution, the oxidation step is preferably performed in a light-shielded condition. Decomposition of the formed triazolinedione compound can be prevented by carrying out the oxidation step in a light-shielded condition.

Note that when an oxidizing agent that does not generate acid as a by-product is used in the oxidation step, water may be generated as a by-product in some cases. For example, when the above-mentioned iodosobenzene (PIO) is used as the oxidizing agent, water is produced as a by-product. When water is present in the reaction system, the formed triazolidinedione compound may be hydrolyzed or cause a side reaction. Therefore, it is preferable to perform the oxidation step while removing water produced as a by-product.

The method of removing water is not particularly limited, and for example, a method of removing water by dry distillation using a solvent that azeotropes with water and a method of performing reaction while allowing a dehydrating agent such as molecular sieves or magnesium sulfate to coexist, etc. can be mentioned. Among these methods, the method of carrying out reaction while allowing a dehydrating agent to coexist is preferable from a viewpoint of the reaction temperature being able to be set to a relatively low temperature. The used amount of a dehydrating agent allowed to coexist may be appropriately determined in consideration of dehydrating efficiency of the dehydrating agent and an amount of water produced by the reaction.

### (Concentration of Triazolinedione Solution)

In the contacting step in the inventive method for producing a solid triazolinedione compound, the triazolinedione solution may be concentrated before the triazolinedione solution is contacted with a hydrocarbon-based poor solvent having 5 to 15 carbon atoms. Triazolidinedione compounds often have low solubility in organic solvents and tend to require a large amount of a used organic solvent in order to rapidly proceed the oxidation step. Therefore, from a viewpoint of obtaining a solid triazolinedione compound in a high yield, it is preferable to concentrate the triazolinedione solution before the triazolinedione solution is contacted with a hydrocarbon-based poor solvent having 5 to 15 carbon atoms.

The degree of concentration is not particularly limited, but it is preferable that the degree be such that the triazolinedione compound starts precipitation. For example, as the concentration of the triazolinedione compound, it is preferable to concentrate the triazolinedione compound to 100 to 50,000 parts by volume with respect to 100 parts by mass of the theoretical amount of the triazolinedione compound formed in the reaction.

The method of concentrating a triazolinedione solution is not particularly limited, and for example, a method, such as concentration under a reduced pressure or the like, can be exemplified.

### (Hydrocarbon-based Poor Solvents)

The hydrocarbon-based poor solvent used in the contacting step is a hydrocarbon-based poor solvent having 5 to 15 carbon atoms. Here, the "hydrocarbon-based poor solvent" refers to a compound which has a hydrocarbon skeleton, in which it is difficult to dissolve a solid, and in which the solubility of the triazolinedione compound is 0.0005 g/100 mL or less.

Examples of such a hydrocarbon-based poor solvent include hexane, heptane, pentane, cyclopentane, cyclohexane, isohexane, isooctane and decane, etc., and among these, it is preferable to use hexane or heptane from a viewpoint of high crystallinity of the triazolinedione compound and stability to the triazolinedione compound.

### (Contact Conditions)

In the contacting step of the present invention, when contacting a triazolinedione solution with a hydrocarbon-based poor solvent, it is preferable that each of the mass of an aprotic good solvent contained in the triazolinedione solution and the mass of a hydrocarbon-based poor solvent to be contacted with the triazolinedione solution should be set to a specific range with respect to the triazolinedione compound represented by the formula (1), as well as a mass ratio of the aprotic good solvent and the hydrocarbon-based poor solvent should be set to a specific range.

Specifically, the amount of the aprotic good solvent is set to 100 to 200,000 parts by mass with respect to 100 parts by mass of the triazolinedione compound represented by the formula (1) in the triazolinedione solution, and the amount of the hydrocarbon-based poor solvent which is to be contacted with the aprotic good solvent is set to 2,500 to 500,000 parts by mass with respect to 100 parts by mass of the triazolinedione compound contained in the triazolinedione solution. Further, it is desirable that the mass ratio of the aprotic good solvent and the hydrocarbon-based poor solvent be set to 1:0.05 to 1:10, and the triazolinedione solution and the hydrocarbon-based poor solvent be contacted with each other. This promotes crystallization of the triazolinedione compound and minimizes loss into the mother liquid.

More preferably, the triazolinedione solution contains an aprotic good solvent in an amount of 500 to 200,000 parts by mass with respect to 100 parts by mass of the triazolinedione compound represented by the formula (1). Further, it is more preferable that the amount of the above-mentioned hydrocarbon-based poor solvent having 5 to 15 carbon atoms be adjusted to 10,000 to 100,000 parts by mass with respect to 100 parts by mass of the triazolinedione compound represented by the formula (1). Further, it is more preferable that the mass ratio of the aprotic good solvent to the hydrocarbon-based poor solvent be 1:0.5 to 1:10.

In the contacting step, the temperature at which the triazolinedione solution is contacted with the hydrocarbon-based poor solvent having 5 to 15 carbon atoms needs to be in the range of -25 to 30°C, and preferably in the range of -10 to 25°C. The range between -25 and 30°C enables prevention of decomposition of the triazolinedione compound and achieves solidification of the triazolinedione compound.

In the contacting step, the time for contacting the triazolinedione solution with the hydrocarbon-based poor solvent having 5 to 15 carbon atoms is preferably 30 minutes to 24 hours, and more preferably 30 minutes to 17 hours.

In the contacting step, it is necessary to perform the contact of a triazolinedione solution with a hydrocarbon-based poor solvent having 5 to 15 carbon atoms in a light-shielded condition. Decomposition of the formed triazolinedione compound can be prevented by carrying out the oxidation step in a light-shielded condition.

### <Methods for producing Triazolinedione Compounds>

The inventive novel method for producing a triazolinedione compound is a method for producing a triazolinedione compound by oxidizing a triazolidinedione compound, the method comprising the step of oxidizing the triazolidinedione compound represented by the following formula (2) using an oxidizing agent that does not generate acid as a by-product to obtain the triazolinedione compound represented by the following formula (1): 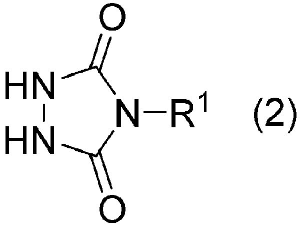 wherein R¹ is an organic group, 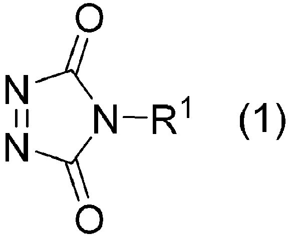 wherein R¹ is an organic group.

### [Oxidation Step]

The reaction solution obtained in the oxidation step in the inventive novel method for producing a triazolinedione compound can be used as the triazolinedione solution in the inventive method for producing a solid triazolinedione compound.

### (Reaction Scheme)

A representative reaction scheme for the oxidation step in the inventive novel method for producing a triazolinedione compound is indicated below. The following is an example of the present invention, and the present invention is not limited to the following.

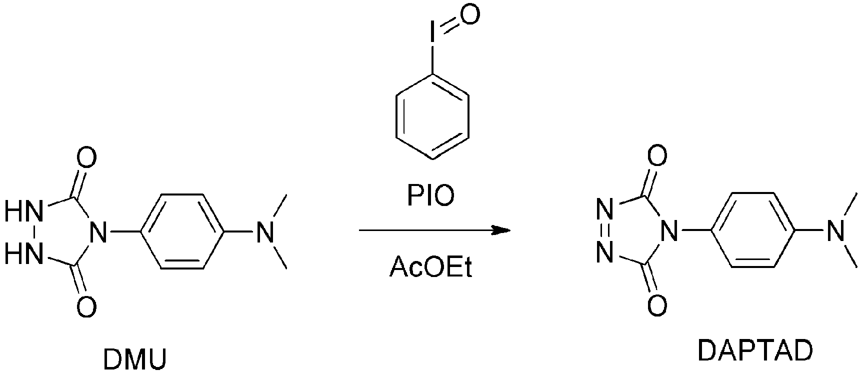

### (Triazolidinedione Compounds)

The triazolidinedione compound of the formula (2) as a starting material in the oxidation step of the inventive novel method for producing a triazolinedione compound is the same as the triazolidinedione compound which is a preferable raw material in the inventive method for producing a solid triazolinedione compound, when a reaction solution obtained by the oxidation step of the triazolidinedione compound is used as the triazolinedione solution.

Specifically, the compound of the formula (2) as a starting material in the oxidation step is a triazolidinedione compound containing a urazole group, that is, a triazolidinedione compound having a 1,2,4-triazolidine-3,5-dione group.

As R¹ in the formula (2), a group selected from the group consisting of a phenyl group, nitrogen-containing heterocyclic groups and alkyl groups, with the phenyl group, the nitrogen-containing heterocyclic groups and the alkyl groups optionally containing a disubstituted amino group substituted with the same or different alkyl groups, aralkyl groups or aryl groups, with the alkyl groups, aralkyl groups or aryl groups optionally containing an oxygen atom or a nitrogen atom; a nitro group; an azido group; an alkoxy group; a halogen group; an alkylthio group; a sulfonyl group; a phosphate group; a carboxyl group; an ester group; a nitrile group; an amide group; a ferrocenyl group; or a quinoxalinyl group having a substituent is preferred.

More preferably, R¹ in the formula (2) is a group selected from the group consisting of a substituted or unsubstituted phenyl, methyl and ethyl groups.

Furthermore, R¹ in the formula (2) is particularly preferably a group selected from the group consisting of a phenyl group, a methyl group, a 2-(6,7-dimethoxy-4-methyl-3-oxo-3,4-dihydroquinoxalinyl) ethyl group, a 4-nitrophenyl group, a ferrocenylmethyl group, a 4-dimethylaminophenyl group and 4-dimethylaminomethylphenyl group.

Therefore, as the triazolinedione compound represented by the formula (1), which is obtained by oxidizing the triazolidinedione compound represented by the formula (2), using the inventive novel method for producing a triazolinedione compound, compounds selected from the group consisting of 4-phenyl-1,2,4-triazoline-3,5-dione (PTAD), 4-methyl-1,2,4-triazoline-3,5-dione (MTAD), 4-[2-(6,7-dimethoxy-4-methyl-3-oxo-3,4-dihydroquinoxalinyl)ethyl]-1,2,4-triazoline-3,5-dione (DMEQTAD), 4-(4-nitrophenyl)-1,2,4-triazoline-3,5-dione (NPTAD), 4-ferrocenylmethyl-1,2,4-triazoline-3,5-dione (FMTAD), 4-(6-quinolyl)-1,2,4-triazoline-3,5-dione (QTAD), 4-(4'-diethylaminophenyl)-1,2,4-triazoline-3,5-dione (DEAPTAD), 4-(4'-dimethylaminophenyl)-1,2,4-triazoline-3,5-dione (DAPTAD) and 4-(4'-dimethylaminomethylphenyl)-1,2,4-triazoline-3,5-dione are particularly preferred. These compounds may be Cookson type derivatizing agents.

Furthermore, most preferably, R¹ in the formula (2) is a 4-dimethylaminophenyl group or a 4-dimethylaminomethylphenyl group. Namely, as the triazolidinedione compound represented by the formula (2), 4-(4'-dimethylaminophenyl)-1,2,4-triazolidine-3,5-dione (DMU) is the most preferred, and as the triazolinedione compound obtained by the inventive novel method for producing the triazolinedione compound, 4-(4'-dimethylaminophenyl)-1,2,4-triazoline-3,5-dione (DAPTAD), or 4-(4'-dimethylaminomethylphenyl)-1,2,4-triazoline-3,5-dione are the most preferred.

### (Oxidizing agent)

In the oxidation step in the inventive novel process for producing the triazolinedione compound, the oxidizing agent to be used is an oxidizing agent that does not generate acid as a by-product.

For example, when a triazolidinedione compound is oxidized to obtain a triazolinedione compound by using iodobenzene diacetate (PIDA), which is conventionally known as an oxidizing agent and which is described in Non-Patent Documents 1 to 4, acetic acid is generated as a by-product. Moreover, when washing is performed with an alkaline aqueous solution in order to remove the by-product acetic acid, the obtained triazolinedione compound is decomposed.

Therefore, in the oxidation step in the inventive novel method for producing the triazolinedione compound, an oxidizing agent that does not generate acid as a by-product is used. Examples of the oxidizing agent that does not generate acid as a by-product include a super-valent iodine compound represented by the following formula: 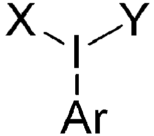 wherein X and Y together represent one oxygen atom, or X and Y are the same or different groups selected from the group consisting of a hydroxy group, an alkoxy group, an acylamino group, a tosylamino group and a halogen group; and Ar represents a phenyl group, a heterocyclic group, a phenyl group substituted with an alkyl group, an alkoxy group and a halogen group, etc.

In the oxidation step in the inventive novel method for producing a triazolinedione compound, it is preferable to use, for instance, iodosobenzene (PIO), which is a compound described in the above-mentioned reaction scheme.

The amount of the oxidizing agent used is preferably 1.0 to 5.0 mol equivalents, and more preferably 1.0 to 2.0 mol equivalents, with respect to the triazolidinedione compound as a raw material. The range of 1.0 to 5.0 mol equivalents is preferable because it facilitates removal of the oxidizing agent present after the reaction and decomposition products thereof.

Note that when an oxidizing agent that does not generate acid as a by-product is used in the oxidation step, water may be generated as a by-product in some cases. For example, when the above-mentioned iodosobenzene (PIO) is used as the oxidizing agent, water is produced as a by-product. When water is present in the reaction system, the formed triazolidinedione compound may be hydrolyzed or cause a side reaction. Therefore, it is preferable to perform the oxidation step while removing water produced as a by-product.

The method of removing water is not particularly limited, and for example, a method of removing water by dry distillation using a solvent that azeotropes with water and a method of performing a reaction while allowing a dehydrating agent such as molecular sieves or magnesium sulfate to coexist, etc. can be mentioned. Among these methods, the method of carrying out a reaction while allowing a dehydrating agent to coexist is preferable from a viewpoint of the reaction temperature being able to be set to a relatively low temperature. The used amount of a dehydrating agent allowed to coexist may be appropriately determined in consideration of dehydrating efficiency of the dehydrating agent and an amount of water produced by the reaction.

### (Aprotic Good Solvent)

In the oxidation step in the inventive novel method for producing a triazolinedione compound, a triazolidinedione compound is oxidized in an aprotic good solvent to obtain the triazolinedione compound.

As the aprotic good solvent, the same solvents as those used in the triazolinedione solution in the above-mentioned method for producing a solid triazolinedione compound can be used. Specifically, the aprotic good solvent is preferably at least one selected from the group consisting of esters, halogen-containing hydrocarbons, aromatic hydrocarbons, ketones, amides, alkylnitriles, dialkyl ethers and ureas.

Further, examples include ethyl acetate, methyl acetate, butyl acetate, isopropyl acetate, tetrahydrofuran (THF), 2-methyltetrahydrofuran (2-methyl THF), 1,4-dioxane, t-butyl methyl ether, 1,2-dimethoxyethane, diglyme, acetone, diethyl ketone, methyl ethyl ketone, methylene chloride, chloroform, carbon tetrachloride, 1,2-dichloroethane, chlorobenzene, toluene, xylene, dimethylformamide (DMF), dimethylacetamide (DMA), dimethyl sulfoxide (DMSO), N-methylpyrrolidone (NMP) and 1,3-dimethyl-2-imidazolydinone (DMI), etc. These may be used singly or as mixed solvents.

Among these, it is preferable to use in the oxidation step in the novel method for producing a triazolinedione compound at least one selected from the group consisting of methylene chloride, acetonitrile, 1,2-dimethoxyethane, toluene and ethyl acetate, from a viewpoint of acceleration of the oxidation reaction, stability against the oxidation reaction and stability of the triazolinedione compound to be produced.

### (Oxidation Conditions)

In the oxidation step in the inventive novel method for producing a triazolinedione compound, the aprotic good solvent is blended so that the amount of the aprotic good solvent is 100 to 200,000 parts by mass with respect to 100 parts by mass of the triazolidinedione compound represented by the formula (2). More preferably, the aprotic good solvent is blended so that the amount of the aprotic good solvent is 100 to 100,000 parts by mass with respect to 100 parts by mass of the triazolidinedione compound. A solvent amount in this range is preferable because triazolinedione can be dissolved without heating.

The temperature in the oxidation step is preferably in the range of -10 to 50°C, and more preferably in the range of 0 to 30°C.

The reaction time in the oxidation step is preferably 1 minute to 48 hours, more preferably 10 minutes to 17 hours.

The oxidation step is preferably performed in a light-shielded condition. Decomposition of the formed triazolinedione compound can be prevented by carrying out the oxidation step in a light-shielded condition.

### <Method of Quantification of Triazolinedione Compound>

A triazolinedione compound represented by the formula (1), to which the inventive production method of a solid triazolinedione compound can be applied, or which is a solid triazolinedione compound of the present invention, or which can be obtained by the inventive novel production method of a triazolinedione compound, can be reacted with a diene compound of the following formula (3) to obtain an ene-compound represented by the following formula (4), and then the amount thereof can be analyzed.

Examples of the method of analysis include, but are not limited to, high performance liquid chromatography (HPLC) analysis.

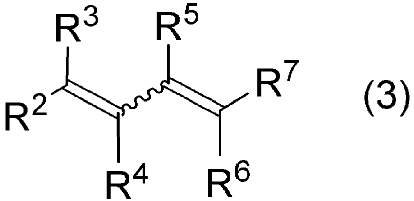

Wherein R², R³, R⁴, R⁵, R⁶ and R⁷ are the same or different alkyl groups, aralkyl groups, phenyl groups, or heterocyclic groups having 1 to 100 carbon atoms, with the alkyl groups, the aralkyl groups, the phenyl groups, or the heterocyclic groups optionally containing an oxygen atom, a nitrogen atom, a sulfur atom or a phosphorus atom.

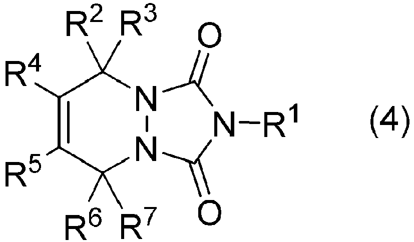

Wherein R¹ is the same as in formula (1) above, and R², R³, R⁴, R⁵, R⁶, R⁷ and R⁸ are the same as in formula (3) above.

The diene compound represented by the formula (3) is not particularly limited, but, for example, trans,trans-diphenylbutadiene (TTB) is preferable because TTB is easily industrially available as a high-purity diene compound, an ene-compound obtained by reacting TTB with triazolidinedione has high stability, and TTB is highly sensitive in high performance liquid chromatography (HPLC) analysis of the ene-compound.

When the triazolinedione compound represented by the formula (1) is 4-(4'-dimethylaminophenyl)-1,2,4-triazoline-3,5-dione (DAPTAD) and the diene compound represented by the formula (3) is trans,trans-diphenylbutadiene (TTB), the ene-compound obtained by reacting is cis-2-(4-dimethylaminophenyl-5,8-diphenyl-1H-[1,2,4]triazolo[1,2-a]pyridazin-1,3(2H)-dione (DAPTAC) represented by the following formula (5). The reaction scheme is also shown below.

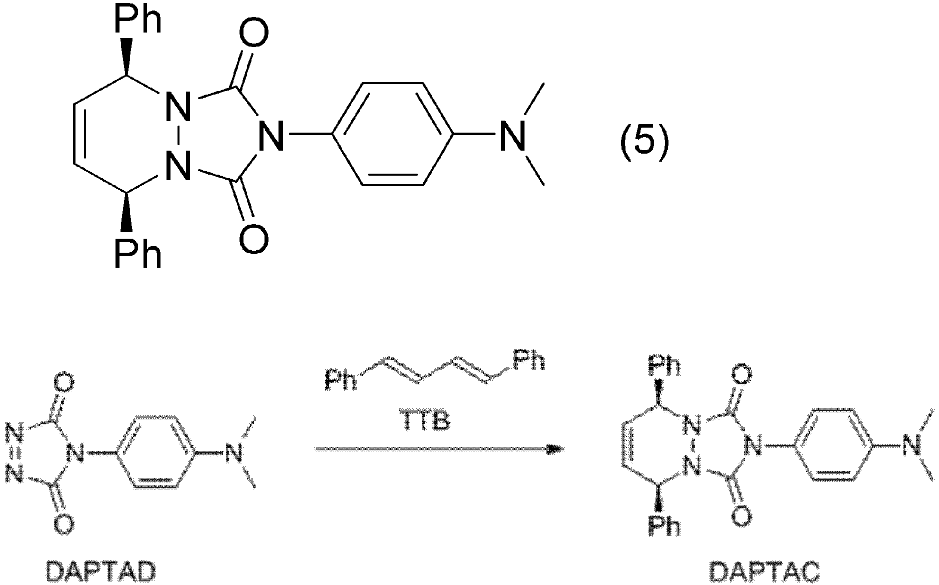

Performing high performance liquid chromatography (HPLC) analysis with regard to DAPTAC in a solution enables DAPTAT, which is a raw material for DAPTAC, to be quantified.

### EXAMPLES

Subsequently, the Examples of the present invention are described below, but the present invention is not limited to these Examples.

### <Reference Example 1>

### Method using Ethyl Acetate as Solvent

A suspension of 0.030 g (0.136 mmol) of 4-(4'-dimethylaminophenyl)-1,2,4-triazolidine-3,5-dione (DMU) in ethyl acetate (30 mL) was obtained. To the obtained suspension, 0.044 g (0.137 mmol) of iodobenzene diacetate (PIDA) was added in a light-shielded condition under a nitrogen stream, oxidation of DMU was performed by stirring at room temperature for 2 hours, and an ethyl acetate solution in which 4-(4'-dimethylaminophenyl)-1,2,4-triazoline-3,5-dione (DAPTAD) was dissolved was obtained.

### [Assay Yield]

To the resulting reaction solution, 0.028 g (0.136 mmol) of trans,trans-diphenylbutadiene (TTB) was added and the reaction solution was stirred at 20°C for 1 hour to synthesize cis-2-(4-dimethylaminophenyl-5,8-diphenyl-1H-[1,2,4]triazolo[1,2-a]pyridazin-1,3(2H)-dione (DAPTAC).

With regard to the DAPTAC in the solution, high performance liquid chromatography (HPLC) analysis was performed under the following conditions to quantify the DAPTAD as a raw material for DAPTAC, and finally, the assay yield of the DAPTAD was calculated. The assay yield of the DAPTAD was 58%.

### (Analysis Conditions)

Sample concentration: 50% (the reaction solution was diluted with the same weight of THF and measured)
Injection volume: 1.0 µL
Wavelength: 254 nm
Flow rate: 1.0 mL/min.
Mobile phase: 0 to 15 min. (CH₃CN:water = 50:50 to CH₃CN:water = 100:0)
15 to 20 min. (CH₃CN:water = 100:0)
Column temperature: 30°C
Filler: XBridge C18 5 µm (4.6 x 150)
Retention time: DMU: 2.1 min.
DAPTAC: 7.2 min.
TTB: 11.4 min.

### [Reaction Scheme]

The reaction scheme performed in Reference Example 1 is shown below.

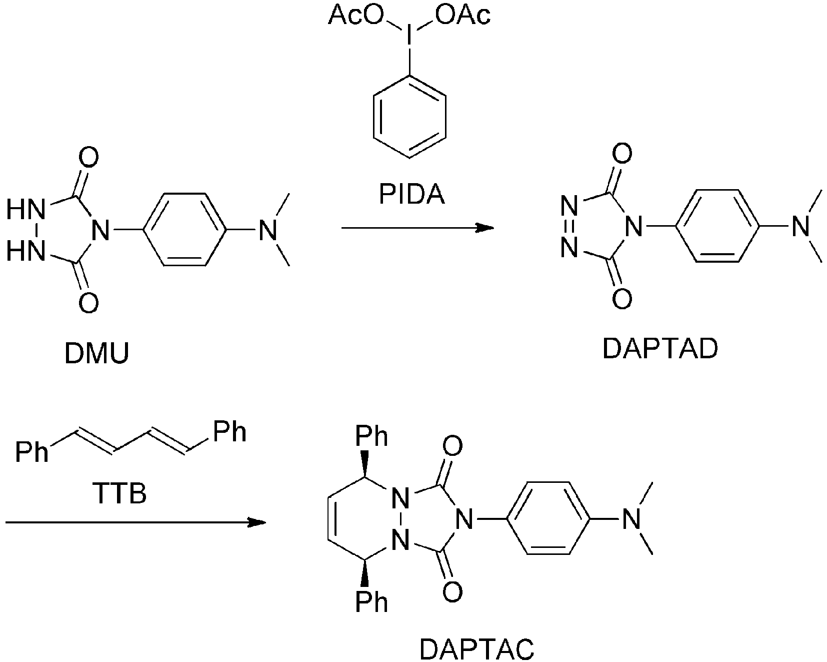

### [Isolation of DAPTAC]

A reaction solution containing synthesized DAPTAC was washed with 5% sodium bicarbonate water, followed by washing with water, then the reaction solution was concentrated under reduced pressure, and the concentrated residue was purified using a silica gel column (eluting solvent: ethyl acetate). After concentrating fractions containing a product under reduced pressure, the concentrated residue was heated and dispersed in 10 mL of ethyl acetate, and cooled to room temperature to precipitate DAPTAC crystals. The precipitated crystals were filtered, washed with hexane, dried under reduced pressure and a solid DAPTAC was obtained. The obtained solid DAPTAC weighed 0.55 g and the yield was 58%.

### [Physical Property Evaluation]

The results of various analyses of the obtained solid-state cis-2-(4-dimethylaminophenyl)-5,8-diphenyl-1H-[1,2,4]triazolo[1,2-a]pyridazine-1,3(2H)-dione (DAPTAC) are shown below. Mp: 174 to 177°C
IR (KBr): 1772 and 1700 cm⁻¹
¹H-NMR (CDCl₃) : δ 7.10-7.75(m, 12H)
6.50-6.85(m, 2H)
6.00(s, 2H)
5.51(s, 2H)
2.90(s, 6H)

### <Reference Example 2>

### Method using Methylene Chloride as Solvent

DAPTAC was synthesized in the same manner as in Reference Example 1, except that methylene chloride was used as the solvent, and the assay yield was calculated by high performance liquid chromatography (HPLC). The assay yield was 71.7%.

### <Reference Example 3>

### Method using Chloroform (Stabilizer: Amylene) as Solvent

DAPTAC was synthesized in the same manner as in Reference Example 1, except that methylene chloride (stabilizer: amylene) was used as the solvent, and the assay yield was calculated by high performance liquid chromatography (HPLC). The assay yield was 23.7%.

### <Reference Example 4>

### Method using Acetonitrile as Solvent

DAPTAC was synthesized in the same manner as in Reference Example 1, except that acetonitrile was used as the solvent, and the assay yield was calculated by high performance liquid chromatography (HPLC). The assay yield was 75.8%.

### <Reference Example 5>

### Methods using Dimethyl sulfoxide (DMSO) as Solvent

DAPTAC was synthesized in the same manner as in Reference Example 1, except that dimethyl sulfoxide (DMSO) was used as the solvent, and the assay yield was calculated by high performance liquid chromatography (HPLC) assay. The assay yield was 11.7%.

### <Reference Example 6>

### Method using Dimethylformamide (DMF) as Solvent

DAPTAC was synthesized in the same manner as in Reference Example 1, except that dimethylformamide (DMF) was used as the solvent, and the assay yield was calculated by high performance liquid chromatography (HPLC) assay. The assay yield was 53.4%.

### <Reference Example 7>

### Method using Tetrahydrofuran (THF) as Solvent

DAPTAC was synthesized in the same manner as in Reference Example 1, except that tetrahydrofuran (THF) was used as the solvent, and the assay yield was calculated by high performance liquid chromatography (HPLC) assay. The assay yield was 58.6%.

### <Reference Example 8>

### Method using 1,2-Dimethoxyethane as Solvent

DAPTAC was synthesized in the same manner as in Reference 1, except that 1,2-dimethoxyethane was used as the solvent, and the assay yield was calculated by high performance liquid chromatography (HPLC). The assay yield was 76.9%.

### <Reference Example 9>

### Method using Toluene as Solvent

DAPTAC was synthesized in the same manner as in Reference Example 1, except that toluene was used as the solvent, and the assay yield was calculated by high performance liquid chromatography (HPLC). The assay yield was 63.1%.

### <Reference Example 10>

### Method using Ethyl Acetate as Solvent

DAPTAC was synthesized in the same manner as in Reference Example 1, except that ethyl acetate was used as the solvent, and the assay yield was calculated by high performance liquid chromatography (HPLC). The assay yield was 52.1%.

### <Example 1>

### [Preparation of Triazolinedione Solution (Oxidation of Triazolidinedione Compound)]

A suspension in which 0.50 g (2.23 mmol) of 4-(4'-dimethylaminophenyl)-1,2,4-triazolidine-3,5-dione (DMU) was suspended in ethyl acetate (500 mL) was obtained. To the obtained suspension, 0.73 g (2.26 mmol) of iodobenzene diacetate (PIDA) was added in a light-shielded condition under a nitrogen stream, the mixture was stirred at 20°C for 2 hours to oxidize DMU, and an ethyl acetate solution in which 4-(4'-dimethylaminophenyl)-1,2,4-triazoline-3,5-dione (DAPTAD) was dissolved was obtained. The resulting ethyl acetate solution was red.

### [Contacting Step]

The resulting ethyl acetate solution was filtered, and then the mother liquid was concentrated under reduced pressure to about 40 mL where crystals of 4-(4'-dimethylaminophenyl)-1,2,4-triazoline-3,5-dione (DAPTAD) were precipitated out. To the ethyl acetate solution concentrated under reduced pressure condition, 200 mL of hexane was added in a light-shielded condition and stirred at room temperature (about 20°C) for 2 hours, to crystallize 4-(4'-dimethylaminophenyl)-1,2,4-triazoline-3,5-dione (DAPTAD) out.

The ethyl acetate-hexane mixed solution in which 4-(4'-dimethylaminophenyl)-1,2,4-triazoline-3,5-dione (DAPTAD) was crystallized was filtered, the obtained solid was washed with hexane and dried under reduced pressure at room temperature, and a solid DAPTAD was obtained. The resulting solid DAPTAD was purple crystals and weighed 0.35 g. The yield was 70%.

### [Physical Property Evaluation]

The results of various analyses of the obtained solid 4-(4'-dimethylaminophenyl)-1,2,4-triazoline-3,5-dione (DAPTAD) are shown below.
Mp: 187 to 200°C (dec)
UV: λmax 540 nm
IR(KBr) : 1764, 1749 cm⁻¹
¹H-NMR (CDCl₃): δ 7.00-7.50 (m, 2H)
6.50-7.00(m, 2H)
3.00 (s, 6H)

### <Example 2>

### [Preparation of Triazolinedione Solution (Oxidation of Triazolidinedione Compound)]

A suspension in which 0.30 g (1.34 mmol) of 4-(4'-dimethylaminophenyl)-1,2,4-triazolidine-3,5-dione (DMU) was suspended in 300 mL of 1,2-dimethoxyethane (DME) was obtained. To the obtained suspension, 0.44 g (1.34 mmol) of iodobenzene diacetate (PIDA) was added in a light-shielded condition under a nitrogen stream, stirred at 20°C for 2 hours to oxidize DMU, and a 1,2-dimethoxyethane solution in which 4-(4'-dimethylaminophenyl)-1,2,4-triazoline-3,5-dione (DAPTAD) was dissolved was obtained. The reaction solution was red.

### [Contacting Step]

The resulting 1,2-dimethoxyethane solution was filtered, and then the mother liquid was concentrated under reduced pressure to about 24 mL where crystals of 4-(4'-dimethylaminophenyl)-1,2,4-triazoline-3,5-dione (DAPTAD) were precipitated out. To the 1,2-dimethoxyethane solution concentrated under reduced pressure, 120 mL of hexane was added in a light-shielded condition and stirred at room temperature (about 20°C) for 2 hours, to crystallize 4-(4'-dimethylaminophenyl)-1,2,4-triazoline-3,5-dione (DAPTAD) out.

The 1,2-dimethoxyethane-hexane mixed solution in which 4-(4'-dimethylaminophenyl)-1,2,4-triazoline-3,5-dione (DAPTAD) was crystallized was filtered, the obtained solid was washed with hexane and dried under reduced pressure at room temperature, and a solid DAPTAD was obtained. The resulting solid DAPTAD was purple crystals and weighed 0.24 g. The yield was 82%.

### [Assay Yield]

0.03 g of the obtained solid DAPTAD was dissolved in 30 mL of 1,2-dimethoxyethane, 28 mg (0.14 mmol) of trans,trans-diphenylbutadiene(TTB) was added to this, and stirred at 20°C for 1 hour to synthesize cis-2-(4-dimethylaminophenyl-5,8-diphenyl-1H-[1,2,4]triazolo[1,2-a]pyridazin-1,3(2H)-dione(DAPTAC).

With regard to the DAPTAC in the solution, high performance liquid chromatography (HPLC) was carried out under the following conditions to quantify the DAPTAD as a raw material for DAPTAC, and finally, the assay yield of DAPTAD was calculated. The assay yield of the DAPTAD was 48%.

### (Analysis Conditions)

Sample concentration: 0.05%
Injection volume: 1.0 µL
Wavelength: 254 nm
Flow rate: 1.0 mL/min.
Mobile phase: 0 to 15 min. (CH₃CN:water = 50:50 to CH₃CN: water = 100:0)
15 to 20 min. (CH₃CN:water = 100:0)
Column temperature: 30°C
Filler: X Bridge C18 5 µm (4.6 x 150)
Retention time: DMU: 2.1 min.
DAPTAC: 7.2 min.
TTB: 11.4 min.

### <Example 3>

### Example of performing the Oxidation Step at 50°C

### [Preparation of Triazolinedione Solution (Oxidation of Triazolidinedione Compound)]

The oxidation reaction of DMU was carried out in the same manner as in Example 2, except that the oxidation reaction of DMU was carried out by stirring at 50°C for 2 hours, and a 1,2-dimethoxyethane solution in which 4-(4'-dimethylaminophenyl)-1,2,4-triazoline-3,5-dione (DAPTAD) was dissolved was obtained.

### [Contacting Step]

A solid DAPTAD was obtained in the same manner as in the contacting step of Example 2. The resulting solid DAPTAD was purple crystals and weighed 0.099 g. The yield was 33%.

### [Assay Yield]

The obtained solid DAPTAD of 0.099 g was dissolved in 300 mL of 1,2-dimethoxyethane, 276 mg (1.34 mmol) of TTB was added thereto, and the mixture was stirred at 20°C for 30 minutes to synthesize DAPTAC. An assay yield of the DAPTAD was calculated by performing high performance liquid chromatography (HPLC) analysis in the same manner as in Example 2. The assay yield was 33.1%.

### <Comparative Example 1>

### Example of concentrating Reaction Solution to Dryness to obtain Solid

### [Preparation of Triazolinedione Solution (Oxidation of Triazolidinedione Compound)]

The oxidation of DMU was performed in the same manner as in Example 2, and a 1,2-dimethoxyethane solution in which 4-(4'-dimethylaminophenyl)-1,2,4-triazoline-3,5-dione (DAPTAD) was dissolved was obtained.

### [Concentration of Reaction Solution to Dryness]

The obtained 1,2-dimethoxyethane solution was completely concentrated under a reduced pressure at 30°C, 10 times volume of hexane was added to the concentrated residue, the mixture was stirred at 20°C overnight to precipitate crystals, crystals were filtered, and a solid was obtained.

### [Assay Yield]

The resulting solid was dissolved in 3 mL of dimethyl sulfoxide (DMSO), 276 mg (1.34 mmol) of TTB was added, stirred at 20°C for 30 minutes, and then analyzed by HPLC in the same manner as in Example 2. The assay yield of the DAPTAD was 17.2%.

### <Comparative Example 2>

### Example of setting Crystallization Temperature (Contact Temperature) in the Contacting Step to 50°C

### [Preparation of Triazolinedione Solution (Oxidation of Triazolidinedione Compound)]

The oxidation reaction of DMU was carried out in the same manner as in Example 2, and a 1,2-dimethoxyethane solution in which 4-(4'-dimethylaminophenyl)-1,2,4-triazoline-3,5-dione (DAPTAD) was dissolved was obtained.

### [Contacting Step]

A solid substance was obtained by performing a contacting step in the same manner as in Example 2, except that the stirring temperature was set to 50°C. The resulting solid was dark red crystals.

### [Assay Yield]

The resulting solid was dissolved in 300 mL of 1,2-dimethoxyethane, 276 mg (1.34 mmol) of trans,trans-diphenylbutadiene (TTB) was added thereto, the mixture was stirred at 20°C for 30 minutes, and a TTB adduct was obtained.

The assay yield of the DAPTAD was calculated by performing high performance liquid chromatography (HPLC) analysis on the TTB-adduct in the solution in the same manner as in Example 2. The assay yield of the DAPTAD was 0%.

### <Comparative Example 3>

### Example in which the oxidation step and the contacting step were performed without shielding light

### [Preparation of Triazolinedione Solution (Oxidation of Triazolidinedione Compound)]

The oxidation of DMU was performed in the same manner as in Example 2, except that light was not shielded, to obtain a 1,2-dimethoxyethane solution.

### [Contacting Step]

A solid substance was obtained by performing a contacting step in the same manner as in Example 2, except that light was not shielded. The resulting solid was dark red crystals.

### [Assay Yield]

The resulting solid was dissolved in 300 mL of 1,2-dimethoxyethane, 276 mg (1.34 mmol) of trans,trans-diphenylbutadiene (TTB) was added thereto, the mixture was stirred at 20°C for 30 minutes, and a TTB adduct was obtained.

The assay yield of the DAPTAD was calculated by performing high performance liquid chromatography (HPLC) analysis on the TTB-adduct in the solution in the same manner as in Example 2. The assay yield of the DAPTAD was 0%.

### <Comparative Example 4>

### Example of using Bis(trifluoroacetoxy)iodobenzene as Oxidizing Agent

### [Preparation of Triazolinedione Solution (Oxidation of Triazolidinedione Compound)]

A suspension in which 0.30 g (1.34 mmol) of DMU was suspended in ethyl acetate (300 mL) was obtained. To the obtained suspension, 0.59 g (1.37 mmol) of bis(trifluoroacetoxy)iodobenzene was added in a light-shielded condition, and the mixture was stirred at 20°C for 3 hours to attempt oxidation reaction of the DMU. The resulting ethyl acetate solution was red.

### [Contacting Step]

After the obtained ethyl acetate solution was filtered, the mother liquid was concentrated under reduced pressure to a solution amount of 20 g. To the ethyl acetate solution concentrated under reduced pressure, 50 mL of hexane was added in a light-shielded condition and stirred at room temperature (about 20°C) for 1 hour, to crystallize a solid.

The ethyl acetate-hexane mixed solution in which the solid was crystallized was filtered, the obtained solid was washed with hexane and dried under reduced pressure at room temperature for 1 hour, and a solid was obtained in an amount of 0.23 g. The resulting solid was analyzed by ¹H-NMR and found to be not a target substance (DAPTAD).

### <Comparative Example 5>

### Example of using Calcium Hypochlorite as Oxidizing Agent

### [Preparation of Triazolinedione Solution (Oxidation of Triazolidinedione Compound)]

A suspension in which 0.50 g (2.23 mmol) of DMU was suspended in ethyl acetate (500 mL) was obtained. To the obtained suspension, 0.65 g (4.55 mmol) of calcium hypochlorite was added in a light-shielded condition, and the mixture was stirred at 20°C for 3 hours to attempt oxidation reaction of DMU. The resulting ethyl acetate solution was yellow.

### [Contacting Step]

After the obtained ethyl acetate solution was filtered, the mother liquid was concentrated under reduced pressure to a solution volume of 20 mL. To the ethyl acetate solution concentrated under reduced pressure, 50 mL of hexane was added in a light-shielded condition and stirred at room temperature (about 20°C) for 10 minutes, to crystallize a solid out.

The ethyl acetate-hexane mixed solution in which the solid was crystallized was filtered, the obtained solid was washed with hexane and dried under reduced pressure at room temperature, and a solid was obtained in an amount of 0.42 g. The resulting solid was analyzed by ¹H-NMR and found to be not a target substance (DAPTAD).

### <EXAMPLE 4>

### [Preparation of Triazolinedione Solution (Oxidation of Triazolidinedione Compound)]

A suspension in which 0.30 g (1.34 mmol) of 4-(4'-dimethylaminophenyl)-1,2,4-triazolidine-3,5-dione (DMU) was suspended in ethyl acetate (300 mL) was obtained. To the obtained suspension, 0.30 g (1.36 mmol) of iodosobenzene (PIO) was added in a light-shielded condition under a nitrogen stream, the mixture was stirred at 25°C for 4 hours to oxidize DMU, and an ethyl acetate solution in which 4-(4'-dimethylaminophenyl)-1,2,4-triazoline-3,5-dione (DAPTAD) was dissolved was obtained. The reaction liquid was red.

### [Contacting Step]

The obtained ethyl acetate solution was filtered, and then the mother liquid was concentrated under reduced pressure at 25°C to about 15 mL. To the ethyl acetate solution concentrated under reduced pressure condition, 500 mL of hexane was added in a light-shielded condition and stirred at room temperature (about 20°C) for 10 hours, to crystallize a solid out.

The supernatant liquid was removed by decantation, then a hexane-insoluble part was concentrated under reduced pressure, and a solid DAPTAD was obtained. The solid DAPTAD obtained weighed 0.22 g and the yield was 74%.

### [Physical Property Evaluation]

The results of various analyses of the obtained solid 4-(4'-dimethylaminophenyl)-1,2,4-triazoline-3,5-dione (DAPTAD) are shown below.
UV: λmax 540 nm
IR (KBr) : 1764, 1749 cm⁻¹
¹H-NMR (CDCl₃): δ 7.00-7.50 (m, 2H)
6.50-7.00 (m, 2H)
3.00 (s, 6H)

### <EXAMPLE 5>

### [Preparation of Triazolinedione Solution (Oxidation of Triazolidinedione Compound)]

A suspension in which 0.30 g (1.69 mmol) of 4-phenyl-1,2,4-triazolidine-3,5-dione (DMU) was suspended in ethyl acetate (30 mL) was obtained. To the obtained suspension, 0.37 g (1.68 mmol) of iodosobenzene (PIO) was added in a light-shielded condition under a nitrogen stream, the mixture was stirred at 25°C for 4 hours, and an ethyl acetate solution in which 4-phenyl-1,2,4-triazoline-3,5-dione was dissolved was obtained. The reaction scheme is shown below.

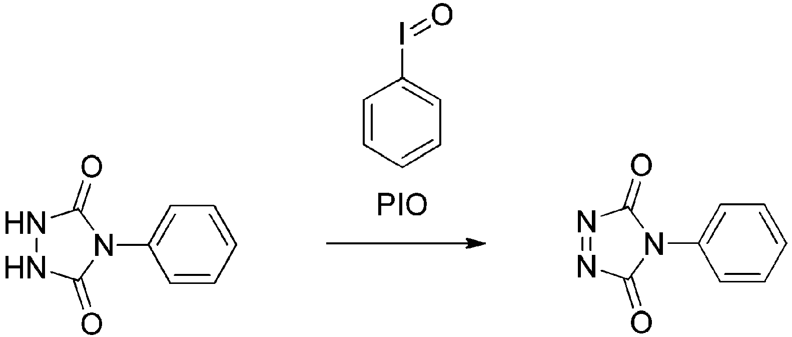

### [Contacting Step]

After the obtained ethyl acetate solution was filtered, 100 mL of hexane was added to the filtrate in a light-shielded condition, and the filtrate was stirred at room temperature (about 20°C) for 1 hour to crystallize 4-phenyl-1,2,4-triazoline-3,5-dione out.

The ethyl acetate-hexane mixed solution in which the 4-phenyl-1,2,4-triazoline-3,5-dione was crystallized was filtered, the obtained solid was washed with hexane and dried under reduced pressure at room temperature, and a solid 4-phenyl-1,2,4-triazoline-3,5-dione was obtained. The resulting solid weighed 192 mg and the yield was 65%.

### [Physical Property Evaluation]

The results of various analyses of the obtained solid 4-phenyl-1,2,4-triazoline-3,5-dione are shown below.
Mp: 165 to 175°C (dec)
IR (KBr) : 1745 cm⁻¹
¹H-NMR (CDCl₃) : δ 6.70-8.00 (m, 5H)

### <EXAMPLE 6>

### [Preparation of Triazolinedione Solution (Oxidation of Triazolidinedione Compound)]

A suspension in which 0.195 g (1.69 mmol) of 4-methyl-1,2,4-triazolidine-3,5-dione was suspended in ethyl acetate (30 mL) was obtained. To the obtained suspension, 0.37g (1.68 mmol) of iodosobenzene (PIO) was added in a light-shielded condition under a nitrogen stream, the mixture was stirred at 25°C for 4 hours to perform oxidation, and an ethyl acetate solution in which 4-methyl-1,2,4-triazoline-3,5-dione was dissolved was obtained. The reaction scheme is shown below.

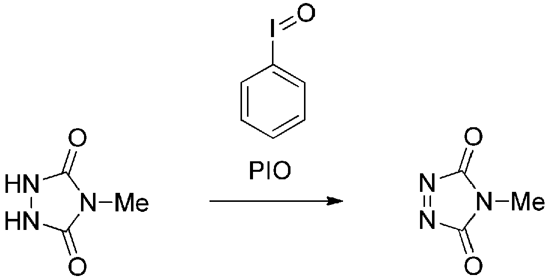

### [Contacting Step]

The obtained ethyl acetate solution was filtered, then 100 mL of hexane was added to the filtrate in a light-shielded condition and stirred at room temperature (about 20°C) for 1 hour, to crystallize 4-methyl-1,2,4-triazoline-3,5-dione out.

The ethyl acetate-hexane mixed solution in which 4-methyl-1,2,4-triazoline-3,5-dione was crystallized was filtered, the obtained solid was washed with hexane and dried under reduced pressure at room temperature, and a solid 4-methyl-1,2,4-triazoline-3,5-dione was obtained. The resulting solid weighed 134 mg and the yield was 70%.

### [Physical Property Evaluation]

The results of various analyses of the obtained solid 4-methyl-1,2,4-triazoline-3,5-dione are shown below.
Mp: 97 to 99°C (dec)
IR (KBr) : 1760 cm⁻¹
¹H-NMR (CDCl₃) : δ 3.10 (s, 3H)

### <EXAMPLE 7>

### [Preparation of Triazolinedione Solution (Oxidation of Triazolidinedione Compound)]

A suspension in which 0.50 g (2.27 mmol) of 4-(4'-dimethylaminophenyl)-1,2,4-triazolidine-3,5-dione (DMU) was suspended in 500 mL of ethyl acetate from which dissolved oxygen had been removed by nitrogen-bubbling was obtained. To the obtained suspension, 0.50 g (2.27 mmol) of iodosobenzene (PIO) and 2.0 g of magnesium sulfate (anhydrous) as a dehydrating agent were added in a light-shielded condition under a nitrogen stream and stirred at 20°C for 2 hours to oxidize DMU and an ethyl acetate solution in which 4-(4'-dimethylaminophenyl)-1,2,4-triazoline-3,5-dione (DAPTAD) was dissolved was obtained. The resulting ethyl acetate solution was red.

### [Contacting Step]

The resulting ethyl acetate solution was filtered and poured into 250 mL of heptane from which dissolved oxygen had been removed by nitrogen-bubbling. The mother liquid was subjected to solvent substitution from ethyl acetate to heptane by concentrating under reduced pressure to about 30 mL where crystals of 4-(4'-dimethylaminophenyl)-1,2,4-triazoline-3,5-dione (DAPTAD) were crystallized out. To the heptane solution concentrated under reduced pressure, 250 mL of heptane, from which dissolved oxygen had been removed by nitrogen-bubbling, was further added in a light-shielded condition and stirred at room temperature (about 20°C) for 2 hours, to crystallize 4-(4'-dimethylaminophenyl)-1,2,4-triazoline-3,5-dione (DAPTAD) out.

The heptane solution in which 4-(4'-dimethylaminophenyl)-1,2,4-triazoline-3,5-dione (DAPTAD) was crystallized was filtered, the obtained solid was washed with heptane and dried under reduced pressure at room temperature, and a solid DAPTAD was obtained. The resulting solid DAPTAD was purple crystals and weighted 0.39 g. The yield was 78%.

### [Assay Yield]

0.0209 g (0.096 mmol) of the obtained solid DAPTAD was dissolved in 10 g of acetonitrile, 0.0487 g (0.24 mmol) of trans,trans-1,4-diphenyl-1,3-butadiene (TTB) was added to this and stirred at 20°C for 1 hour, and cis-2-(4-dimethylaminophenyl)-5,8-diphenyl-1H-[1,2,4]triazolo[1,2-a]pyridazin-1,3(2H)-dione(DAPTAC) was synthesized. The assay yield of the DAPTAD was calculated by carrying out high performance liquid chromatography (HPLC) analysis in the same manner as in Example 2. The assay yield of the DAPTAD was 56%.

### <EXAMPLE 8>

### [Preparation of Triazolinedione Solution (Oxidation of Triazolidinedione Compound)]

A suspension in which 2.00 g (9.08 mmol) of 4-(4'-dimethylaminophenyl)-1,2,4-triazolidine-3,5-dione (DMU) was suspended in 2,000 mL of ethyl acetate from which dissolved oxygen had been removed by nitrogen-bubbling was obtained. To the obtained suspension, 2.00 g (9.08 mmol) of iodosobenzene (PIO) and 8.0 g of magnesium sulfate (anhydrous) as a dehydrating agent were added in a light-shielded condition under a nitrogen stream and stirred at 20°C for 2 hours to oxidize DMU, and an ethyl acetate solution in which 4-(4'-dimethylaminophenyl)-1,2,4-triazoline-3,5-dione (DAPTAD) was dissolved was obtained. The resulting ethyl acetate-solution was red.

### [Contacting Step]

The resulting ethyl acetate solution was filtered and poured into 1,000 mL of heptane from which dissolved oxygen had been removed by nitrogen-bubbling. The mother liquid was subjected to solvent substitution from ethyl acetate to heptane by concentrating under reduced pressure to about 120 mL where crystals of 4-(4'-dimethylaminophenyl)-1,2,4-triazoline-3,5-dione (DAPTAD) were crystallized out. To the heptane solution concentrated under reduced pressure, 1,000 mL of heptane (from which dissolved oxygen had been removed by nitrogen-bubbling) was further added in a light-shielded condition and stirred at room temperature (about 20°C) for 2 hours, to crystallize 4-(4'-dimethylaminophenyl)-1,2,4-triazoline-3,5-dione (DAPTAD) out.

The heptane solution in which 4-(4'-dimethylaminophenyl)-1,2,4-triazoline-3,5-dione (DAPTAD) was crystallized was filtered, the obtained solid was washed with heptane and dried under reduced pressure at room temperature, and a solid DAPTAD was obtained. The resulting solid DAPTAD was purple crystals and weighed 1.63 g. The yield was 82%.

### [Assay Yield]

0.0214 g (0.098 mmol) of the obtained solid DAPTAD was dissolved in 10 g of acetonitrile, 0.0423 g (0.21 mmol) of trans,trans-1,4-diphenyl-1,3-butadiene (TTB) was added to this and stirred at 20°C for 1 hour to synthesize cis-2-(4-dimethylaminophenyl)-5,8-diphenyl-1H-[1,2,4]triazolo[1,2-a]pyridazin-1,3(2H)-dione(DAPTAC). The assay yield of the DAPTAD was calculated by carrying out high performance liquid chromatography (HPLC) analysis in the same manner as in Example 2. The assay yield of the DAPTAD was 61%.

## Claims

1. A method for producing a solid triazolinedione compound, the method comprising contacting a triazolinedione solution in which a triazolinedione compound represented by the following formula (1) is dissolved in an aprotic good solvent with a hydrocarbon-based poor solvent having 5 to 15 carbon atoms in a light-shielded condition at -25 to 30°C: 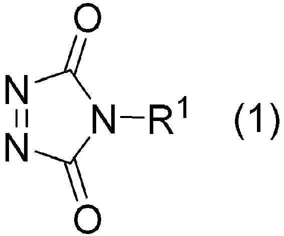 wherein R¹ is an organic group.

2. The method for producing a solid triazolinedione compound according to claim 1, wherein the aprotic good solvent is at least one type selected from the group consisting of esters, halogen-containing hydrocarbons, aromatic hydrocarbons, ketones, amides, alkylnitriles, dialkyl ethers and ureas.

3. The method for producing a solid triazolinedione compound according to claim 1 or 2, wherein in the contacting step, the triazolinedione solution and the hydrocarbon-based poor solvent are contacted with each other by setting an amount of the aprotic good solvent in the triazolinedione solution to 100 parts by mass to 200,000 parts by mass relative to 100 parts by mass of the triazolinedione compound represented by the formula (1);
setting an amount of the hydrocarbon-based poor solvent to 2,500 to 500,000 parts by mass relative to 100 parts by mass of the triazolinedione compound represented by the formula (1); and
setting a mass ratio of the aprotic good solvent to the hydrocarbon-based poor solvent to 1:0.05 to 1:10.

4. The method for producing a solid triazolinedione compound according to any one of claims 1 to 3, wherein the triazolinedione solution is obtained through an oxidation step of oxidizing a triazolidinedione compound represented by the following formula (2) using an oxidizing agent that does not generate acid as a by-product to obtain the triazolinedione compound represented by the formula (1): 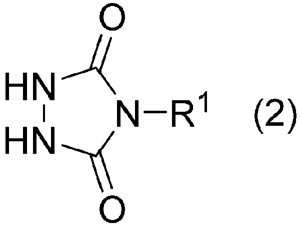 wherein R¹ is an organic group.

5. The method for producing a solid triazolinedione compound according to claim 4, wherein the oxidizing agent is iodosobenzene.

6. The method for producing a solid triazolinedione compound according to claim 4 or 5, wherein the oxidation step is performed while removing water produced as a by-product.

7. The method for producing a solid triazolinedione compound according to any one of claims 4 to 6, wherein the oxidation step is performed in a light-shielded condition.

8. A solid triazolinedione compound represented by the following formula (1): 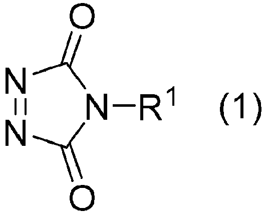 wherein R¹ is a group selected from the group consisting of (a), (b) and (c) below:
(a) a substituted phenyl group comprising a disubstituted amino group or a disubstituted aminoalkyl group, with the disubstituted amino group or the disubstituted aminoalkyl group being substituted with the same or different alkyl groups, aralkyl groups or aryl groups, with the alkyl groups, the aralkyl groups or the aryl groups optionally comprising an oxygen atom or a nitrogen atom; a nitro group; an azide group, an alkoxy group; a halogen group; an alkylthio group; a sulfonyl group, a phosphate group; a carboxyl group; an ester group; a nitrile group; an amide group; a ferrocenyl group or a substituted quinoxalinyl group;
(b) a nitrogen-containing heterocyclic group optionally comprising a disubstituted amino group substituted with the same or different alkyl groups, aralkyl groups or aryl groups, with the alkyl groups, the aralkyl groups or the aryl groups optionally comprising an oxygen atom or a nitrogen atom; a nitro group; an azide group; an alkoxy group; a halogen group; an alkylthio group; a sulfonyl group; a phosphate group; a carboxyl group; an ester group; a nitrile group; an amide group; a ferrocenyl group or a substituted quinoxalinyl group; and
(c) an alkyl group optionally comprising a disubstituted amino group substituted with the same or different alkyl groups, aralkyl groups or aryl groups, with the alkyl groups, the aralkyl groups or the aryl groups optionally comprising an oxygen atom or a nitrogen atom; a nitro group; an azide group; an alkoxy group; a halogen group; an alkylthio group; a sulfonyl group; a phosphate group; a carboxyl group; an ester group; a nitrile group; an amide group; a ferrocenyl group or a substituted quinoxalinyl group.

9. The solid triazolinedione according to claim 8, wherein R¹ in the formula (1) is a 4-dimethylaminophenyl group or a 4-dimethylaminomethylphenyl group.

10. A method for producing a triazolinedione compound, the method comprising oxidizing a triazolidinedione compound to obtain the triazolinedione compound,
wherein the method comprises an oxidation step of oxidizing a triazolidinedione compound represented by the following formula (2) by using an oxidizing agent that does not generate acid as a by-product to obtain the triazolinedione compound represented by the following formula (1) : 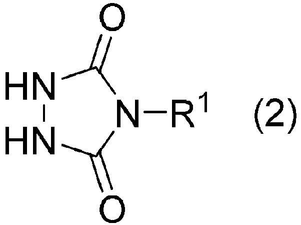 wherein R¹ is an organic group, 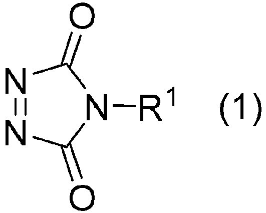 wherein R¹ is an organic group.
